# EUROPÄISCHE PATENTSCHRIFT

(11) **EP 2 303 197 B1**
(45) Veröffentlichungstag und Bekanntmachung des Hinweises auf die Patenterteilung: **29.08.2012**
(21) Anmeldenummer: 09749490.0
(22) Anmeldetag: 19.05.2009
(51) Int. Cl.: A61F 2/68, A61F 2/76

(54) **ORTHOPÄDIETECHNISCHE EINRICHTUNG**
ORTHOPEDIC TECHNICAL DEVICE
DISPOSITIF ORTHOPÉDIQUE

(30) Priorität: 20.05.2008 DE 102008024746
(43) Veröffentlichungstag der Anmeldung: 06.04.2011
(73) Patentinhaber: Otto Bock HealthCare GmbH, 37115 Duderstadt (DE)
(72) Erfinder: HAHN, Tanja, 37115 Duderstadt (DE); ALBRECHT-LAATSCH, Erik, 37077 Göttingen (DE)
(74) Vertreter: Plöger, Jan Manfred
(86) Internationale Anmeldenummer: PCT/DE2009/000688
(87) Internationale Veröffentlichungsnummer: WO 2009/140948

(56) Entgegenhaltungen:
- WO-A-2008/033852
- DE-A1- 3 909 672
- DE-A1- 19 859 931

## Beschreibung

Die Erfindung betrifft eine orthopädietechnische Einrichtung für die unteren Extremitäten mit (a) mindestens einer Aufnahme für eine Extremität, (b) einem Sensor und (c) einer mit dem Sensor verbundenen elektrischen Auswerteeinheit zum Verarbeiten von Daten des Sensors.

Aus der DE 39 09 672 A1 ist eine Oberschenkelprothese gemäß dem Oberbegriff von Anspruch 1 bekannt. Nachteilig an einer derartigen Oberschenkelprothese ist, dass sie aufwändig eingelernt werden muss. Wird der Winkelsensor beispielsweise auf der falschen Seite angebaut, so ist ein Einlernen nicht möglich.

Aus der WO 99/44547 ist eine Steuereinheit für eine Prothese bekannt, die eine dynamisch anpassbare Kniebewegungssteuereinheit umfasst, die die Flexion und/oder die Extension des Knies in Abhängigkeit einer Variabilität eines kinematischen Parameters verändert. Nachteilig ist auch hieran, dass diese orthopädietechnische Einrichtung aufwändig eingelernt werden muss und bei grober Fehlmontage des Sensors nicht funktioniert.

Aus der DE 10 2005 051 496 A1 ist ein Verfahren zur Durchführung einer Funktionsanalyse einer künstlichen Extremität bekannt. Dieses Verfahren setzt nach einer ursprünglichen Einrichtung der orthopädietechnischen Einrichtung ein und verbessert die ablaufenden Verfahren. In der Druckschrift ist keine Lehre enthalten, wie auch bei einer Fehlmontage des Sensors schnell eingelenkt werden kann.

Aus der DE 34 05 081 A1 ist ein Sportschuh für Laufdisziplinen bekannt. Nachteilig an diesem Sportschuh ist, dass er zur Verwendung als orthopädietechnische Einrichtung nicht geeignet ist.

Eine derartige orthopädietechnische Einrichtung kann beispielsweise eine Orthese sein, die dazu dient, Patienten mit Restmuskelaktivität, deren Muskelkraft für normales Gehen nicht ausreicht, das Gehen zu ermöglichen. Orthopädietechnische Einrichtungen wie eine solche Orthese umfassen Komponenten, die in Massenverfahren hergestellt werden können. Einzelne Komponenten müssen jedoch aneinander montiert und, beispielsweise von einem Orthopädietechniker, individuell an den Patienten angepasst werden, der die orthopädietechnische Einrichtung tragen soll.

Nachteilig an bekannten orthopädietechnischen Einrichtungen ist, dass das Einrichten auf den jeweiligen Patienten aufwändig ist. Es kann zudem zu Fehlmontagen von Komponenten kommen. Das senkt die Akzeptanz der orthopädietechnischen Einrichtung beim Patienten und führt zu Kosten und der Gefahr einer falschen Einstellung aufgrund von Fehlbedienungen. Dies kann dann dazu führen, dass bei Benutzung der orthopädietechnischen Einrichtung Orthese beispielsweise eine Arretier- und/oder Dämpfvorrichtung zu spät oder zu früh aktiviert wird, was die Wahrscheinlichkeit eines Unfalls erhöht.

Der Erfindung liegt die Aufgabe zugrunde, eine orthopädietechnische Einrichtung schneller oder zuverlässiger einrichtbar zu gestalten.

Die Erfindung löst das Problem durch eine gattungsgemäße orthopädietechnische Einrichtung, bei der die elektrische Auswerteeinheit eingerichtet ist, um ein Verfahren mit den Schritten (i) Schalten in einen Lernmodus, (ii) Ermitteln mindestens eines System-Kennwerts, (iii) Speichern des System-Kennwerts, (iv) Schalten in einen Betriebsmodus und (v) im Betriebsmodus, Optimieren oder Auswählen eines Steueralgorithmus der orthopädietechnische Einrichtung anhand des System-Kennwerts (R, S, N, a_{W,Schwelle}).

Vorteilhaft an dieser orthopädietechnischen Einrichtung ist, dass sie besonders leicht an einen Patienten angepasst werden kann. Vorteilhaft ist zudem die einfache und intuitive Bedienbarkeit der orthopädietechnischen Einrichtung. An die Montage des Sensors an der orthopädietechnischen Einrichtung sind damit nur geringe Anforderungen gestellt, so dass auch weniger qualifiziertes Personal zum Montieren und Anpassen dieser Montage geeignet ist.

Es ist ein weiterer Vorteil, dass der Sensor an einer Vielzahl von Positionen angebracht werden kann, ohne dass seine Funktion beeinträchtigt wird. Das erlaubt es, den Sensor dort anzubringen, wo es den Patienten wenig stört. Das erhöht die Akzeptanz der orthopädietechnischen Einrichtung. Der Sensor kann beispielsweise auch von der Aufnahme beabstandet am Knöchel oder unter der Sohle auf einer kontralateralen Seite angeordnet sein.

Vorteilhaft an dieser orthopädietechnischen Einrichtung ist, dass beispielsweise ein Montagefehler des Orthopädietechnikers das sichere Funktionieren nicht beeinträchtigt.

Im Rahmen der vorliegenden Beschreibung wird unter dem Sensor insbesondere jede Vorrichtung verstanden, die eingerichtet und angeordnet ist, um eine Lage, Richtung, Kraft oder ein Moment zu messen. Unter dem Verarbeiten von Daten des Sensors wird insbesondere verstanden, dass die Daten zum Steuern und/oder Regeln einer Vorrichtung verwendet werden und/oder zur späteren Auswertung gespeichert werden. Beispielsweise kann bei aufrechtem Stand, in dem keine Momentenbelastung vorliegen sollte, der Momentenwert korrigiert werden.

Der Lernmodus könnte auch als Kalibrierfunktion bezeichnet werden. Diese Kalibrierfunktion ist nicht auf eine spätere Korrektur von Sensordaten beschränkt. Alternativ oder additiv ist es möglich, Verläufe zu wählen, die dem ermittelten System-Kennwert zugeordnet sind. Es ist zudem möglich, Verstärkungsfaktoren (Gain), Verschiebungsfaktoren (Offset), Linearitätsfehler oder Schwellenwerte zu ermitteln. Alternativ oder additiv ist es außerdem möglich, patientenabhängige Kennwerte zu ermitteln.

Unter dem Steueralgorithmus wird insbesondere ein Programm verstanden, das in der Auswerteeinheit abgelegt ist und vom Sensor gemessenen Daten einliest und beispielsweise Steuerbefehle an einen Aktuator der orthopädietechnischen Einrichtung ausgibt. Unter dem Merkmal, dass die Steuereinheit Auswerteeinheit ist zum Durchführen der angegebenen Schritte, wird insbesondere verstanden, dass die Auswerteeinheit über einen digitalen Speicher verfügt, in dem Programmkode abgelegt ist. Dieser Programmkode führt dazu, dass die Steuereinheit die angegebenen Schritte automatisch durchführt.

Gemäß einer bevorzugten Ausführungsform ist die elektrische Auswerteeinheit eingerichtet, um ein Verfahren mit den Schritten (i) Schalten in einen Lernmodus, (ii) Ermitteln mindestens eines Montagefehler-Kennwerts, der einen Montagefehler des Sensors kodiert, (iii) Speichern des Montagefehler-Kennwerts, (iv) Schalten in den Betriebsmodus und (v) Korrigieren von Daten des Sensors anhand des mindestens einen Montagefehler-Kennwerts.

Unter einem Montagefehler-Kennwert wird dabei insbesondere eine Größe verstanden, die eine Abweichung zwischen einer Soll-Position und/oder einer Soll-Lage des Sensors und einer Ist-Position bzw. einer Ist-Lage des Sensors kodiert. Die Abweichung beruht beispielsweise darauf, dass der Orthopädietechniker den Sensor nicht an der vorgesehnen Stelle angebracht hat, weil er die korrekte Stelle nicht kannte oder weil ein Anbringen an der korrekten Stelle nicht möglich war. Die Ist-Position ist diejenige Position, die der Sensor einnehmen muss, damit die elektrische Auswerteeinheit ohne weitere Korrekturen korrekt funktioniert.

In einer bevorzugten Ausführungsform ist der Sensor ein Winkelsensor zum Erfassen einer Neigung zur Vertikalen. Unter dem Winkelsensor wird insbesondere jegliche Vorrichtung verstanden, die ausgebildet und angeordnet ist, um ihre Orientierung zur Horizontalen bzw. zur Vertikalen zu erfassen und in Form eines Signals kodiert abzugeben. Beispielsweise handelt es sich bei dem Winkelsensor um einen Schwerkraft-Winkelsensor, der immer den genauen Winkel zum Bezugspunkt Erdmittelpunkt liefert. Dazu besitzt ein solcher Schwerkraft-Winkelsensor eine Loterfassungseinheit.

Der Winkelsensor kann zudem eine elektrische Auswerteeinheit umfassen, die das elektrische Neigungssignal generiert. Der Winkelsensor erfasst seine Neigung zur Vertikalen bzw. Horizontalen, die aber nur dann die eigentlich zu messende Neigung der Aufnahme zur Vertikalen bzw. Horizontalen ist, wenn er korrekt montiert ist oder die Messwerte anhand des Montagefehler-Kennwerts korrigiert sind.

Unter der Arretier- und/oder Dämpfvorrichtung wird insbesondere jede mechanische Vorrichtung verstanden, die zumindest zwei Schaltzustände einnehmen kann, nämlich einen aktivierten und einen gelösten Schaltzustand. Im aktivierten Schaltzustand ist ein Verschwenken der Oberschenkelaufnahme relativ zur Unterschenkelaufnahme arretiert oder durch ein Dämpfelement signifikant erschwert. In der gelösten Stellung ist ein entsprechendes Verschwenken möglich. Die Arretier- und/oder Dämpfvorrichtung kann beispielsweise hydraulisch oder rein mechanisch ausgebildet sein.

Unter dem Merkmal, dass das Neigungssignal in Abhängigkeit von dem Montagefehler-Kennwert ermittelt wird, ist insbesondere zu verstehen, dass Rohmesswerte, die die Neigung des Winkelsensors zur Horizontalen bzw. zur Vertikalen kodieren, unter Verwendung des Montagefehler-Kennwerts so korrigiert werden, dass sie danach die Neigung der Oberschenkelaufnahme zur Horizontalen bzw. zur Vertikalen kodieren. Es ist dabei gleichgültig, ob eine Lage zur Horizontalen oder zur Vertikalen kodiert wird, da beide Angaben eine äquivalente Information enthalten, nämlich die Lage der Oberschenkelaufnahme im Raum. Bei einer herkömmlichen Orthese ist beispielsweise der Winkel, bei dem die Arretier- und/oder Dämpfvorrichtung öffnet bzw. arretiert, fest vorgegeben.

In einer bevorzugten Ausführungsform wird der Montagefehler-Kennwert ermittelt, während die Arretier- und/oder Dämpfvorrichtung die Oberschenkelaufnahme und die Unterschenkelaufnahme relativ zueinander arretiert sind, wobei beide bevorzugt in einer Hyperextensionsstellung arretiert sind. Vorteilhafterweise erlaubt diese Stellung ein besonders gefahrloses und damit komfortables Einstellen der Ansteuereinheit.

Gemäß einer bevorzugten Ausführungsform ist der Montagefehler Kennwert ein Radialfehler-Kennwert, der eine Differenz zwischen einem maximalen Wert und einem minimalem Wert des Neigungssignals kodiert. Wird der Winkelsensor nicht exakt lateral, sondern frontal oder dorsal versetzt an der Oberschenkelaufnahme montiert, so verringert sich die Differenz zwischen dem Maximalwert und dem Minimalwert des Neigungssignals. Durch Messung dieser Differenz und damit anhand des Radialfehler-Kennwerts lässt sich das Neigungssignal auf eine vorgegebene Soll-Differenz normieren.

Alternativ oder additiv umfasst das Verfahren bevorzugt den Schritt eines Ermittelns eines Neigungsfehler-Kennwerts, der den Minimalwert oder den Maximalwert des elektrischen Neigungssignals kodiert, wenn die elektrische Auswerteeinheit sich im Lernmodus befindet, und ein Speichern des Neigungsfehler-Kennwerts in dem Digitalspeicher, wobei im Betriebsmodus der elektrischen Auswerteeinheit das Neigungssignal abhängig von dem Neigungsfehler-Kennwert ermittelt wird. Hierunter ist zu verstehen, dass sich das Neigungssignal ändert, wenn sich der Neigungsfehler Kennwert ändert. Beispielsweise ist der Neigungsfehler-Kennwert ein Normierungsfaktor in einer mathematischen Berechnungsformel.

Alternativ oder additiv wird ein Seitenfehler-Kennwert ermittelt, der ein Vorzeichen einer Änderung des Neigungssignals bei einem Vorschwingen der Oberschenkelaufnahme kodiert und der Seitenfehler-Kennwert in dem Digitalspeicher gespeichert, wobei im Betriebsmodus das Neigungssignal in Abhängigkeit von dem Seitenfehler-Kennwert ermittelt wird. Unter dem Vorschwingen der Oberschenkelaufnahme wird dabei eine Schwenkbewegung in der Sagittalebene in frontale Richtung verstanden. Hierdurch kann der Montagefehler erkannt werden, der darin besteht, dass der Winkelsensor auf der falschen Seite der Oberschenkelaufnahme angeordnet wurde. Das heißt, dass beispielsweise ein für eine linke Oberschenkelaufnahme vorgesehener Winkelsensor auf eine rechte Oberschenkelaufnahme montiert worden ist.

Die orthopädietechnische Einrichtung soll verhindern, dass der Patient zu einem falschen Zeitpunkt im Knie einknickt und dadurch stürzt. Es ist daher wichtig, dass die Arretier- und/oder Dämpfvorrichtung sicher geschlossen ist, wenn ein Einknicken droht. Andererseits soll die Arretier- und/oder Dämpfvorrichtung dann geöffnet sein, wenn das Bein nach vorne durchschwingt, um ein angenehmes Tragen zu ermöglichen. Diese Anforderungen werden besonders gut erfüllt, wenn die elektrische Auswerteeinheit der gattungsgemäßen Orthese eingerichtet ist, um ein Verfahren mit den Schritten (i) Ermitteln des Maximalwerts des Neigungssignals, (ii) Ermitteln eines Schwellenwerts, der kleiner ist als der Maximalwert, (iii) Senden eines Öffnungsbefehls an die Arretier- und/oder Dämpfvorrichtung, wenn das Neigungssignal den Schwellenwert unterschreitet, (iv) Erfassen, ob die Arretier- und/oder Dämpfvorrichtung gelöst ist, und (v) verneinendenfalls Verringern des Schwellenwerts, (vi) des Ermittelns des Schwellenwerts bis zum etwaigen Verringern des Schwellenwerts, bis die Arretier- und/oder Dämpfvorrichtung gelöst ist, und (vii) Speichern des letzten Schwellenwerts durchzuführen. Der letzte Schwellenwert ist dabei ein Montagefehler-Kennwert. Die genannten Schritte werden im Lernmodus durchgeführt. Im Betriebsmodus arretiert bzw. löst die Arretier- und/oder Dämpfvorrichtung, wenn dieser Schwellenwert unter- bzw. überschritten wird.

Der erste Schwellenwert wird beispielsweise dadurch ermittelt, dass die Differenz zwischen dem Maximalwert und dem Minimalwert ermittelt wird. Diese Differenz wird anschließend in eine Vielzahl von insbesondere gleichgroßen Abschnitten aufgeteilt, beispielsweise in zehn oder mehr Abschnitte. In ersterem Fall entspricht der zehnte Abschnitt dem Maximalwert und der nullte Abschnitt dem Minimalwert. Als erster Schwellenwert wird dann beispielsweise die neunte Stufe festgelegt. Es ist möglich, mehr Stufen als zehn vorzusehen. Es ist auch möglich, die Stufen nicht äquidistant zu wählen.

Ein Schwellenwert, der besonders geeignet ist, um eine angenehm zu tragende Orthese zu erhalten, wird dadurch erzeugt, dass ermittelt wird, ob nach einer vorgegebenen Anzahl an Öffnungsbefehlen und einer zugehörigen Zahl an Schritten des Patienten mit der Orthese die Arretier- und/oder Dämpfvorrichtung gelöst ist. Dabei werden die Schritte des Ermittelns des Schwellenwerts bis zum etwaigen Verringern des Schwellenwerts für einen gleich bleibenden Schwellenwert so lange wiederholt, bis die Arretier- und/oder Dämpfvorrichtung bei der vorgegebenen Anzahl an Öffnungsbefehlen gelöst ist. Erst danach wird der letzte Schwellenwert gespeichert. Es ist dann sichergestellt, dass die Arretier- und/oder Dämpfvorrichtung mit ausreichend hoher Wahrscheinlichkeit stets rechtzeitig löst und arretiert. Der Schwellenwert, der so ermittelt wird, ist ein Maß für die Neigung der Oberschenkelaufnahme bei der die Arretier- und/oder Dämpfvorrichtung öffnet zur Horizontalen.

Eine modularisierbare Orthese wird erhalten, wenn der Winkelsensor ausgebildet ist, um anhand des Montagefehler-Kennwerts bzw. der Montagefehler-Kennwerte aus einem Rohmesswert von einer Loterfassungsvorrichtung eines Schwerkraft-Winkelsensors das Neigungssignal zu berechnen. In diesem Fall kann das Neigungssignal beispielsweise eine direkte Angabe des Neigungswinkels sein, unter dem die Oberschenkelaufnahme zur Horizontalen bzw. zur Vertikalen ausgerichtet ist. Ein derartiges Neigungssignal kann von der elektrischen Auswerteeinheit besonders leicht verwendet werden, um die Arretier- und/oder Dämpfvorrichtung zum Arretieren und zum Lösen anzusteuern.

Bevorzugt ist die elektrische Auswerteeinheit eingerichtet, um ein Verfahren mit dem Schritt Ausgeben eines Signals, insbesondere eines akustischen oder optischen Signals, das einen Takt vorgibt, in dem ein Benutzer der orthopädietechnischen Einrichtung mit der orthopädietechnischen Einrichtung gehen soll, durchzuführen.

Alternativ oder additiv ist die elektrische Auswerteeinheit eingerichtet, eine Rückmeldung über vom Bediener durchzuführende Schritte auszugeben. Diese Rückmeldung kann via Sprachausgabe oder per Bildschirm gegeben werden. Insbesondere kann die Rückmeldung eine Anweisung für einen vom Benutzer durchzuführenden Bewegungsablauf oder ein Gangmuster sein, also beispielsweise die Anweisung langsamer, schneller, gleichmäßiger, über eine Treppe, über eine Rampe oder im Kreis zu gehen.

Die Erfindung löst das Problem zudem durch ein Verfahren zum Einrichten einer erfindungsgemäßen Orthese mit den Schritten (a) Schalten der Orthese in den Lernmodus, (b) Bewegen der Oberschenkelaufnahme in eine Vorwärtsschrittposition, (c) Eingeben einer Statusinformation in die Auswerteeinheit, die den Zustand kodiert, dass die Oberschenkelaufnahme sich in der Vorwärtsschrittposition befindet, so dass von einer elektrischen Auswerteeinheit der Orthese ein Maximal- oder Minimalwert des Neigungssignals ermittelbar ist, (d) Bewegen der Oberschenkelaufnahme in eine Standposition, so dass von einer elektrischen Auswerteeinheit der Orthese, entsprechend der Minimal- oder Maximalwert des Neigungssignals ermittelbar ist, (e) Eingeben einer Statusinformation in die Auswerteeinheit, die den Zustand kodiert, dass die Oberschenkelaufnahme in der Standposition ist, und (f) gegebenenfalls Eingeben einer Statusinformation in die Auswerteeinheit, die den Zustand kodiert, dass eine Eingabe beendet ist.

Im Folgenden wird die Erfindung anhand eines exemplarischen Ausführungsbeispiels näher erläutert. Dabei zeigt
- Figur 1: eine erfindungsgemäße Orthese,
- Figur 2: in drei Teilfiguren 2a, 2b und 2c drei mögliche Montagefehler eines Winkelsensors relativ zur Oberschenkelaufnahme der Orthese gemäß Figur 1,
- Figur 3: die Orthese gemäß Figur 1 in einer Vorwärtsschritt-Position,
- Figur 4: die Orthese in einer Schrittende-Position und
- Figur 5: ein Diagramm, das ein Neigungssignal in Abhängigkeit von der Zeit angibt.

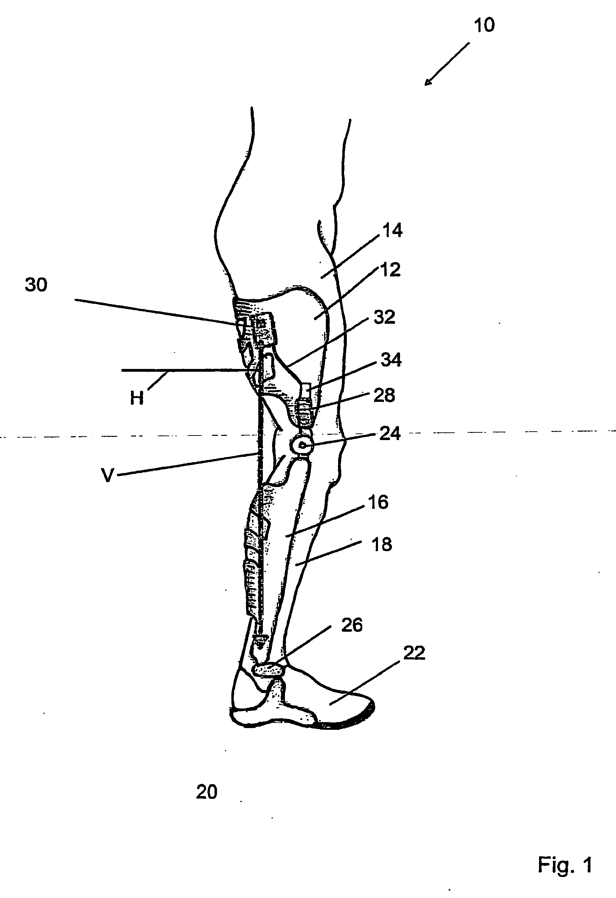

Figur 1 zeigt eine Orthese 10, die eine erste Aufnahme für eine Extremität in Form einer Oberschenkelaufnahme 12 zum Aufnehmen eines Oberschenkels 14, eine zweite Aufnahme in Form einer Unterschenkelaufnahme 16 zum Aufnehmen eines Unterschenkels 18 und eine dritte Aufnahme in Form einer Fußaufnahme 20 zum Aufnehmen eines Fußes 22 umfasst. Die Oberschenkelaufnahme 12 und die Unterschenkelaufnahme 16 sind durch ein Kniegelenk 24 schwenkbar aneinander befestigt. Die Fußaufnahme 20 ist an der Unterschenkelaufnahme 16 durch ein Fußgelenk 26 schwenkbar befestigt.

Das Kniegelenk 24 umfasst eine Arretier- und/oder Dämpfvorrichtung in Form einer Arretiervorrichtung 28, die ausgebildet ist, um aufgrund einer elektrischen Ansteuerung durch ein elektrisches Steuersignal das Kniegelenk 24 durch Arretieren zu aktivieren oder die Arretierung des Kniegelenks 24 zu lösen. Die Arretiervorrichtung 28 steht in elektrischer Verbindung mit einem Sensor in Form eines Winkelsensors 30, der an einem proximalen Ende der Oberschenkelaufnahme 12 lateral angebracht ist.

Der Winkelsensor 30 ist ein Schwerkraft-Winkelsensor, der ausgebildet ist, um ein elektrisches Neigungssignal a_{w} abzugeben. Das Neigungssignal a_{w} kodiert einen Neigungswinkel α_{w}, den der Winkelsensor 30 zu einer Vertikalen V einnimmt. Alternativ kodiert das Neigungssignal a_{w} einen Neigungswinkel, den der Winkelsensor 30 zu einer Horizontalen H einnimmt. Der Neigungswinkel α_{w} stimmt mit dem Neigungswinkel α_{O} zwischen einer Nulllage der Oberschenkelaufnahme 12 und der Vertikalen V genau dann überein, wenn der Winkelsensor 30 korrekt an der Oberschenkelaufnahme 12 montiert worden ist.

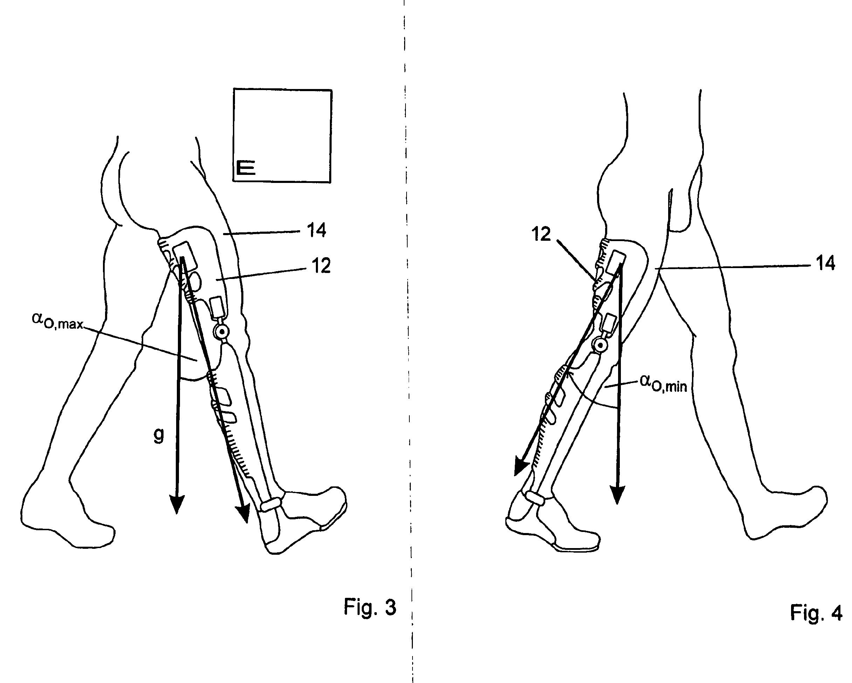

Beim Gehen ändert sich der Neigungswinkel α_{O} zwischen der Oberschenkelaufnahme 12 und der Vertikalen V zwischen einem Maximal-Winkel α_{O,max,} wie er in Figur 3 gezeigt ist, und einem Minimalwinkel α_{O,min,} wie er in Figur 4 gezeigt ist. Der Winkelsensor 30 misst, sofern er korrekt montiert ist, beständig den Neigungswinkel α_{O}, indem er den Neigungswinkel α_{W} misst, und sendet das elektrische Neigungssignal a_{w}, das den Neigungswinkel α_{W} beispielsweise in Form einer Zahl kodiert, an eine schematisch eingezeichnete elektrische Auswerteeinheit 34 der Arretiervorrichtung 28.

Die elektrische Auswerteeinheit 34 vergleicht das Neigungssignal a_{W} in Form der Zahl, die den Neigungswinkel α_{W} kodiert, mit einem Schwellenwert a_{W,Schwelle}, der einen zugehörigen Neigungswinkel α_{W,Schwelle} repräsentiert, und arretiert daraufhin die Arretiervorrichtung 28 oder gibt sie frei. Dadurch ist sichergestellt, dass die Oberschenkelaufnahme 12 und die Unterschenkelaufnahme 16 dann drehstarr miteinander verbunden sind, wenn der Patient sein Bein belastet.

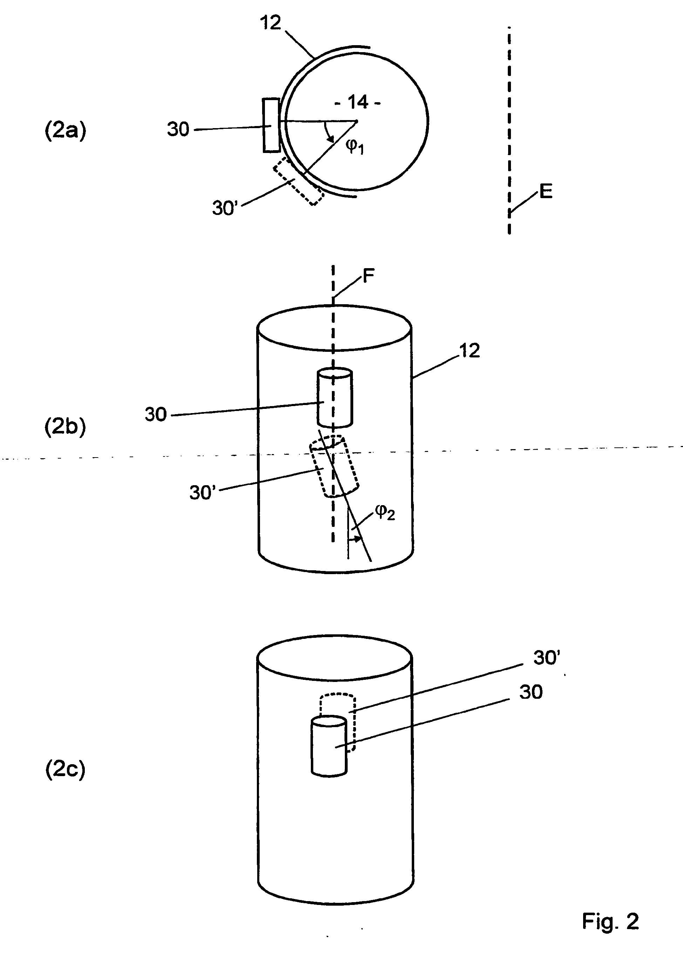

Bei der Montage des Winkelsensors 30 können drei Fehlerquellen unterschieden werden. Eine erste Fehlerquelle ist in der Teilfigur 2a von Figur 2 gezeigt. Während der mit durchgezogener Linie gezeigte Winkelsensor 30 in einer korrekten Position angeordnet ist, nämlich parallel zu einer Sagittalebene E, ist der gestrichelt gezeichnete Winkelsensor 30' um einen Fehlerwinkel ϕ₁ falsch angeordnet. Bei einer Gehbewegung führt das dazu, dass der vom Winkelsensor 30 gemessene Neigungswinkel α_{W} zwischen einem maximalen Neigungswinkel α_{W,max} und einem minimalen Neigungswinkel α_{W,min} schwankt, wobei diese Winkel betragsmäßig kleiner sind als der Maximalwinkel α_{O,max} bzw. α_{O,min} zwischen Oberschenkelaufnahme 12 und der Vertikalen V. Weiter unten wird beschrieben, wie ein Radialfehler-Kennwert R ermittelt werden kann, mit dessen Hilfe der Einfluss des Fehlerwinkels ϕ₁ eliminiert wird.

Teilfigur 2b von Figur 2 zeigt den Fall, dass der Winkelsensor 30 in einer korrekten Postion an der Oberschenkelaufnahme 12 montiert ist, wohingegen der gestrichelt eingezeichnete Winkelsensor 30' um einen Fehlerwinkel ϕ₂ relativ zu einer Frontalebene F anmontiert worden ist. Der Winkelsensor 30 sendet einen Neigungswinkel α_{W}, der sich um den Fehlerwinkel ϕ₂ vom korrekten Neigungswinkel α_{O} unterscheidet. Die Korrektur dieses Fehlers mit Hilfe eines Neigungsfehler-Kennwerts N wird ebenfalls weiter unten beschrieben.

Teilbild 2c von Figur 2 zeigt einen dritten möglichen Fehler, bei dem der Winkelsensor 30' auf der falschen Seite der Oberschenkelaufnahme 12 angeordnet worden ist. Dieser Fehler ließe sich auch als Fehler gemäß Teilfigur 2a auffassen, bei dem der Fehlerwinkel ϕ₁ 180° beträgt.

Die erfindungsgemäße Orthese 10 wird wie im Folgenden beschrieben eingerichtet. Zunächst wird die elektrische Auswerteeinheit 34 in einen Lernmodus geschaltet. Das kann beispielsweise dadurch geschehen, dass ein entsprechender Knopf der Auswerteeinheit 34 gedrückt wird. Alternativ umfasst die Auswerteeinheit 34 beispielsweise einen Infrarot- oder Ultraschallempfänger und es wird ein entsprechendes Infrarot- bzw. Ultraschallsignal mit einer Fernbedienung abgegeben, das die Auswerteeinheit 34 in den Lernmodus schaltet.

Danach wird der Auswerteeinheit 34 eine Statusinformation eingegeben, die kodiert, dass ein Seitenfehler-Erkennungsmodus eingeschaltet werden soll. Nachfolgend wird die Oberschenkelaufnahme 12 in der Sagittalebene E frontal verschwenkt. Das kann beispielsweise dadurch geschehen, dass der Patient seinen Oberschenkel 14 nach vorne schwenkt. In diesem Zustand wird der Auswerteeinheit 34 eine Statusinformation eingegeben, die kodiert, dass die Oberschenkelaufnahme 12 sich in einer Vorwärtsschritt-Position befindet.

Nachfolgend wird die Oberschenkelaufnahme 12 in eine in Figur 1 gezeigte Standposition oder eine in Figur 4 gezeigte Schrittende-Position gebracht, beispielsweise indem der Patient seinen Oberschenkel 14 entsprechend bewegt. Dort angelangt, wird die Auswerteeinheit 34 eine Statusinformation eingegeben, die den Zustand kodiert, dass die Oberschenkelaufnahme 12 in der Standposition bzw. der Schrittende-Position ist. Das Eingeben einer End-Eingabe zeigt der Auswerteeinheit 34 an, dass mit der Auswertung begonnen werden kann.

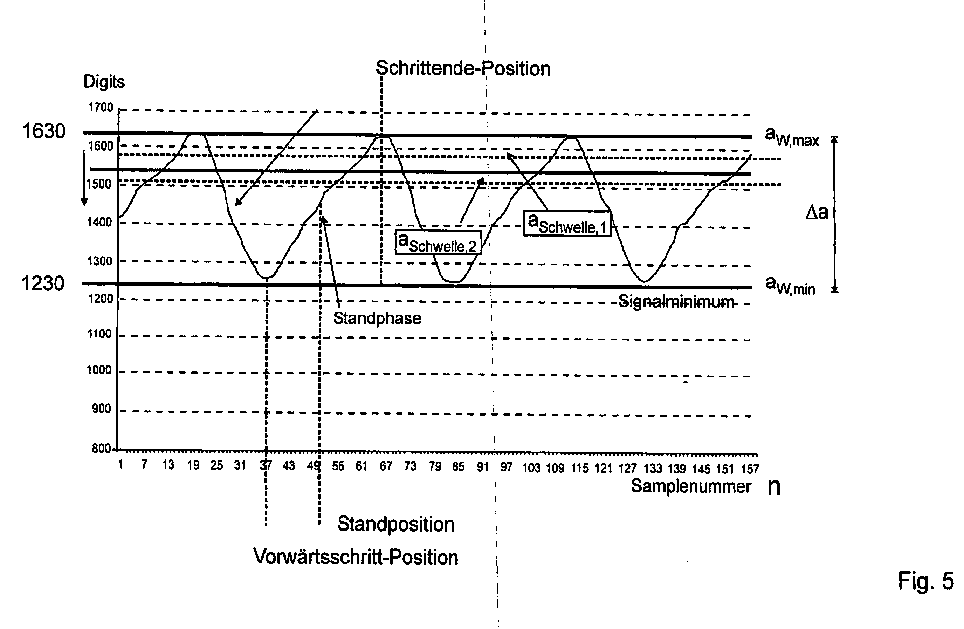

Während der Eingabe hat der Winkelsensor 30 beispielsweise den in Figur 5 gezeigten Verlauf aufgenommen. In Figur 5 ist der Neigungswinkel α_{W} zwischen Winkelsensor und Vertikale in Zähleinheiten (Digits) als a_{W} kodiert angegeben. Die Samplenummer n stellt ein Zeitmaß dar. Bei der Samplenummer 37 wurde die Statusmitteilung eingegeben, dass die Vorwärts-Schrittposition erreicht ist. Bei der Samplenummer 62 wurde eingegeben, dass die Schrittende-Position erreicht ist. Daraus ist zu ersehen, dass der Winkelsensor 30 seitenrichtig angebracht worden ist. Wäre der Winkelsensor auf der falschen Seite angebracht worden, wäre die Vorwärtsschritt-Position bei 1630 Digits gemessen worden und die Schrittende-Position bei 1250. Die Auswerteeinheit 34 speichert daraufhin einen Seitenfehler-Kennwert S in einem digitalen Speicher ab, der den Zustand kodiert, dass kein Seitenfehler vorliegt.

Danach ermittelt die elektrische Auswerteeinheit 34 einen Maximalwert a_{W,max} des Neigungssignals und einen Minimalwert a_{W,min} des Neigungssignals a_{w}, die in die zugehörigen Neigungswinkel α_{W,max} und α_{W,min} umgerechnet werden können. Aus dem Maximalwert a_{W,max} und dem Minimalwert a_{W,min} wird die Differenz Δa berechnet, die im vorliegenden Fall Δa = 1660 - 1250 = 410 beträgt.

Es ist möglich, diese Differenz Δa danach auf einen vorgegebenen Wert zu kalibrieren. Dazu ist es besonders günstig, wenn der Neigungswinkel α_{O} der Oberschenkelaufnahme 12 in der Schrittende-Position (Figur 4) und der Vorwärts-Schritt-Position (Figur 3) auf einen vorgegebenen Wert eingestellt wird, beispielsweise indem ein externer Winkelmesser verwendet wird. Die Differenz Δ a stellt einen möglichen Radialfehler-Kennwert R dar. Der Radialfehler-Kennwert R erlaubt es, das Neigungssignal a_{W} auf eine Soll-Differenz zu normieren.

Nachfolgend speichert die Auswerteeinheit 34 das Signalmaximum a_{W,max} und das Signalminimum a_{W,min} in den digitalen Speicher. Danach wird ein Schwellenwert ermittelt, bei dem die Auswerteeinheit 34 das Kniegelenk 24 arretiert bzw. öffnet.

Dazu wird ein erster Schwellenwert a_{W,Schwelle,1} dadurch ermittelt, dass er als a_{W,Schwelle,1} = a_{W,min} + 0,95*Δa gewählt wird. Der Patient geht nun mit der Orthese und die Auswerteeinheit 34 versucht, das Kniegelenk 24 dann zu öffnen, wenn ein Neigungswinkel α_{Schwelle,1} erreicht ist, der dem Neigungswinkel α_{Schwelle,1} entspricht. Liegt zu diesem Zeitpunkt noch eine Belastung auf dem Knie 24, so kann das Knie 24 nicht gelöst werden, da die Arretiervorrichtung 28 hierzu nicht hinreichend stark ausgebildet ist. Die Auswerteeinheit 34 erfasst, ob das Knie 24 gelöst wurde oder nicht Wurde es nicht gelöst, so wird der Schwellenwert herabgesetzt, beispielsweise auf den Wert a_{W,Schwelle,2} = a_{W,min} + 0,9*Δa.

Dieses Verfahren wird so lange wiederholt, bis die elektrische Auswerteeinheit 34 erstmalig das Kniegelenk 24 zu lösen vermag. Ist das der Fall, wird der jeweilige Schwellenwert konstant gehalten, bis das Kniegelenk während einer Anzahl von n_{G} Gehzyklen, beispielsweise n_{G} = 10 Gehzyklen, jedes Mal korrekt öffnet Ist dies der Fall, wird der entsprechende Schwellenwert a_{W,Schwelle,2} von der Auswerteeinheit 34 in den digitalen Speicher geschrieben. Ist dies nicht der Fall, wird der Schwellenwert a_{W,Schwelle} abgesenkt, bis der korrekte Schwellenwert a_{W,Schwelle} gefunden ist.

Es ist möglich, dass dann, wenn das Kniegelenk sporadisch öffnet, der Schwellenwert um einen kleineren Betrag abgesenkt wird als in dem Fall, dass das Knie 24 nie gelöst werden kann. Ist der Schwellenwert a_{W,Schwelle} gefunden, schaltet die Auswerteeinheit 34 automatisch in den Betriebsmodus. Alternativ oder additiv wird der Auswerteeinheit 34 durch ein entsprechendes Signal, beispielsweise per Tastendruck oder Fernbedienung mitgeteilt, dass der Lernmodus beendet ist. Die Auswerteeinheit 34 schaltet danach in den Betriebsmodus.

Bei korrekter Montage des Winkelsensors 30 und bei natürlichem Gang sind ein Schwellenwert a_{W,normal} und eine Differenz Δ aₙₒᵣₘₐₗ zu erwarten. Der gemäß dem obigen Verfahren ermittelte Schwellenwert a_{W,Schwelle} und die Differenz Δa stellen damit einen Neigungsfehler-Kennwert N bzw. einen Radialfehler-Kennwert R dar.

### Bezugszeichenliste

- 10: Orthese
- 12: Oberschenkelaufnahme
- 14: Oberschenkel
- 16: Unterschenkelaufnahme
- 18: Unterschenkel
- 20: Fußaufnahme
- 22: Fuß
- 24: Kniegelenk
- 26: Fußgelenk
- 28: Arretiervorrichtung
- 30: Winkelsensor
- 32: elektrische Leitung
- 34: Auswerteeinheit

- α_{w}: Neigungswinkel Winkelsensor-Vertikale
- α_{O}: Neigungswinkel Oberschenkelaufnahme-Vertikale
- α_{W,max}: Maximalwinkel
- α_{W,min}: Minimalwinkel
- a_{W}: Neigungssignal, das den Neigungswinkel α_{w} kodiert
- a_{W,Schwelle}: Schwellenwert
- E: Sagittalebene
- F: Frontalebene
- H: Horizontale
- ϕ: Fehlerwinkel
- R: Radialfehler-Kennwert
- S: Seitenfehler-Kennwert
- N: Neigungsfehler-Kennwert
- n_{G}: Zahl der Gehzyklen
- V: Vertikale

## Patentansprüche

1. Orthopädietechnische Einrichtung für die unteren Extremitäten, mit
(a) mindestens einer Aufnahme (12, 16) für eine Extremität (14, 18),
(b) einem Sensor (30) und
(c) einer mit dem Sensor (30) verbundenen elektrischen Auswerteeinheit (34) zum Verarbeiten von Daten des Sensors (30),
**dadurch gekennzeichnet, dass**
(d) die elektrische Auswerteeinheit (34) eingerichtet ist, um ein Verfahren mit den folgenden Schritten auszuführen:
(i) Schalten in einen Lernmodus,
(ii) Ermitteln mindestens eines Montagefehler-Kennwerts (R, S, N, a_{W,Schwelle}), der einen Montagefehler des Sensors (30) kodiert,
(iii) Speichern des Montagefehler-Kennwerts (R, S, N, a_{W,Schwelle}),
(iv) Schalten in einen Betriebsmodus und
(v) im Betriebsmodus Korrigieren von Daten des Sensors anhand des mindestens einen Montagefehler-Kennwerts (R, S, N, a_{W,Schwelle}).

2. Orthopädietechnische Einrichtung nach Anspruch 1, **dadurch gekennzeichnet, dass** der Sensor (30) einen Winkelsensor, Kraftsensor und/oder Momentensensor umfasst.

3. Orthopädietechnische Einrichtung nach einem der vorstehenden Ansprüche, **dadurch gekennzeichnet, dass** der Sensor (30) an der Aufnahme (12, 16) angeordnet ist.

4. Orthopädietechnische Einrichtung nach einem der vorstehenden Ansprüche, **dadurch gekennzeichnet, dass** der Sensor ein Winkelsensor (30) zum Erfassen einer Neigung zur Vertikalen (V) und zum Abgeben eines elektrischen Neigungssignals (a_{w}) ist.

5. Orthopädietechnische Einrichtung nach einem der vorstehenden Ansprüche, **gekennzeichnet durch**
(a) eine Oberschenkelaufnahme (12) zum Befestigen an einem Oberschenkel (14),
(b) eine Unterschenkelaufnahme (16) zum Befestigen an einem Unterschenkel (18) und
(c) eine Arretier- und/oder Dämpfvorrichtung (28) zum lösbaren Arretieren und/oder Dämpfen einer Bewegung von Oberschenkelaufnahme (12) und Unterschenkelaufnahme (16) relativ zueinander.

6. Orthopädietechnische Einrichtung nach einem der vorstehenden Ansprüche, **dadurch gekennzeichnet, dass** die elektrische Auswerteeinheit (34) einen digitalen Speicher besitzt und ausgebildet ist, um im Betriebsmodus die Arretier- und/oder Dämpfvorrichtung (28) in Abhängigkeit von dem Neigungssignal (a_{w}) zum Aktivieren und zum Lösen der Arretier- und/oder Dämpfvorrichtung (28) anzusteuern, und um ein Verfahren mit den folgenden Schritten auszuführen:
(i) Schalten in den Lernmodus,
(ii) Ermitteln des Montagefehler-Kennwerts (R, S, N, a_{W,Schwelle}), der eine Lageabweichung zwischen einer korrekten Position des Winkelsensors (30) und einer tatsächlichen Position des Winkelsensors (30') kodiert,
(iii) Speichern des Montagefehler-Kennwerts (R, S, N, a_{W,Schwelle}) in dem digitalen Speicher, und
(iv) im Betriebsmodus Aktivieren und Lösen der Arretier- und/oder Dämpfvorrichtung (28) anhand des Montagefehler-Kennwerts (R, S, N, a_{W,Schwelle}).

7. Orthopädietechnische Einrichtung nach einem der vorstehenden Ansprüche, **dadurch gekennzeichnet, dass** die elektrische Auswerteeinheit (34) eingerichtet ist zum Aktivieren der Arretier- und/oder Dämpfvorrichtung und zum Ermitteln des Montagefehler-Kennwerts (R, S, N), während die Arretier- und/oder Dämpfvorrichtung (28) die Oberschenkelaufnahme (12) und die Unterschenkelaufnahme (16) relativ zueinander arretiert oder gedämpft sind.

8. Orthopädietechnische Einrichtung nach Anspruch 7, **dadurch gekennzeichnet, dass** die elektrische Auswerteeinheit eingerichtet ist, um ein Verfahren mit den folgenden Schritten auszuführen:
(i) nach dem Schalten in den Lernmodus, Ermitteln eines Montagefehler-Kennwerts in Form eines Neigungsfehler-Kennwerts (N), der den Minimalwert (a_{W,min}) oder den Maximalwert (a_{W,max}) des elektrischen Neigungssignal (a_{w}) kodiert, und
(ii) Speichern des Neigungsfehler-Kennwerts (N) in dem digitalen Speicher,
(iii) wobei im Betriebsmodus die Arretier- und/oder Dämpfvorrichtung (28) anhand des Neigungsfehler-Kennwerts (N) aktiviert und gelöst wird.

9. Orthopädietechnische Einrichtung nach Anspruch 7, **dadurch gekennzeichnet, dass** die elektrische Auswerteeinheit eingerichtet ist, um ein Verfahren mit den folgenden Schritten auszuführen:
(i) Ermitteln eines Montagefehler-Kennwerts in Form eines Seitenfehler-Kennwerts (S), der ein Vorzeichen einer Änderung des Neigungssignals (a_{w}) bei einem Vorschwingen der Oberschenkelaufnahme (12) kodiert, und
(ii) Speichern des Seitenfehler-Kennwerts (S) in dem digitalen Speicher,
(iii) wobei im Betriebsmodus die Arretier- und/oder Dämpfvorrichtung (28) anhand des Seitenfehler-Kennwerts (S) arretiert und gelöst wird.

10. Orthopädietechnische Einrichtung nach einem der Ansprüche 7 bis 8, **dadurch gekennzeichnet, dass** das Ermitteln des Montagefehler Kennwerts (a_{W,Schwelle}) die folgenden Schritte umfasst:
(i) Ermitteln des Maximalwerts (a_{W,max}) des Neigungssignals (a_{w}),
(ii) Ermitteln eines Schwellenwerts (a_{W,Schwelle}), der kleiner ist als der Maximalwert (a_{W,max}).
(iii) Senden eines Öffnungsbefehls an die Arretier- und/oder Dämpfvorrichtung (28), wenn das Neigungssignal (a_{w}) den Schwellenwert (a_{W,Schwelle}) unterschreitet,
(iv) Erfassen, ob die Arretier- und/oder Dämpfvorrichtung (28) gelöst ist, und
(v) verneinendenfalls Verringern des Schwellenwerts (a_{W,Schwelle}),
(vi) Durchführen der Schritte (ii) bis (v), bis die Arretier- und/oder Dämpfvorrichtung (28) gelöst ist, und
(vii) Speichern des Montagefehler-Kennwerts in Form des letzten Schwellenwerts (a_{W,Schwelle}).

11. Orthopädietechnische Einrichtung nach Anspruch 9, **dadurch gekennzeichnet, dass** die elektrische Auswerteeinheit eingerichtet ist, um ein Verfahren mit den folgenden Schritten auszuführen:
- Ermitteln des Minimalwerts (a_{W,min}) des Neigungssignals (a_{w}),
- Speichern des letzten Schwellenwerts (a_{W,Schwelle}), wenn dieser kleiner ist als ein 0,9-faches eines Mittelwerts zwischen Maximalwert (a_{W,max}) und Minimalwert (a_{W,min}).

12. Orthopädietechnische Einrichtung nach Anspruch 9 oder 10, **dadurch gekennzeichnet, dass** die elektrische Auswerteeinheit (34) eingerichtet ist, um ein Verfahren mit den folgenden Schritten auszuführen:
- Ermitteln, ob bei einer vorgegebenen Anzahl (N) an Öffnungsbefehlen die Arretier- und/oder Dämpfvorrichtung (28) gelöst ist, und
Durchführen der Schritte (ii) bis (v) gemäß Anspruch 10 für einen gleich bleibenden Schwellenwert (a_{W,Schwelle}), bis die Arretier- und/oder Dämpfvorrichtung (28) bei der vorgegebenen Anzahl (n_{G}) an Öffnungsbefehlen gelöst ist, bevor der letzte Schwellenwert (a_{W,Schwelle}) gespeichert wird.

13. Verfahren zum Einrichten einer orthopädietechnischen Einrichtung nach einem der vorstehenden Ansprüche, mit den Schritten
(i) Schalten in einen Lernmodus,
(ii) Bewegen der orthopädietechnischen Einrichtung so, dass mindestens ein Montagefehler-Kennwerts (R, S, N, a_{W,Schwelle}) ermittelbar ist,
(iii) Eingeben einer Statusinformation in die Auswerteeinheit (34), die den Zustand kodiert, dass der Montagefehler-Kennwerts (R, S, N, a_{W,Schwelle}) gespeichert werden soll und
(iv) Eingeben einer Statusinformation in die Auswerteeinheit (34), die den Zustand kodiert, dass in den Betriebsmodus geschaltet werden soll.

14. Verfahren nach Anspruch 13, **gekennzeichnet durch** die Schritte
(a) Bewegen der Oberschenkelaufnahme (12) in eine Vorwärtsschrittposition,
(b) Eingeben einer Statusinformation in die Auswerteeinheit (34) , die den Zustand kodiert, dass die Oberschenkelaufnahme (12) sich in der Vorwärtsschrittposition befindet, so dass von einer elektrischen Auswerteeinheit (34) der Orthese (10), ein Maximalwert (a_{W,max}) oder ein Minimalwert (a_{W,min}) des Neigungssignals (a_{w}) ermittelbar ist,
(c) Bewegen der Oberschenkelaufnahme (12) in eine Standposition, so dass von der elektrischen Auswerteeinheit (34) der Orthese (10), entsprechend der Maximalwert (a_{W,max}) oder der Minimalwert (a_{W,min}) Neigungssignals (a_{w}) ermittelbar ist,
(d) Eingeben einer Statusinformation in die Auswerteeinheit, die den Zustand kodiert, dass die Oberschenkelaufnahme (12) in der Standposition ist, und
(e) Eingeben einer Statusinformation in die Auswerteeinheit (34), die den Zustand kodiert, dass eine Eingabe beendet ist.

## Claims

1. An orthopedic technical device for the lower extremities, with
(a) at least one receptacle (12, 16) for an extremity (14, 18),
(b) a sensor (30), and
(c) an electrical evaluation unit (34), connected to the sensor (30), for processing data from the sensor (30),
**characterized in that**
(d) the electrical evaluation unit (34) is configured to carry out a method with the following steps:
(i) switching into a learning mode,
(ii) establishing at least one fitting-error characteristic value (R, S, N, a_{W,Schwelle}) that encodes a fitting error of the sensor (30),
(iii) storing the fitting-error characteristic value (R, S, N, a_{W,Schwelle}),
(iv) switching into an operating mode, and
(v) correcting sensor data with the aid of the at least one fitting-error characteristic value (R, S, N, a_{W,Schwelle}) in the operating mode.

2. The orthopedic technical device as claimed in claim 1, **characterized in that** the sensor (30) comprises an angle sensor, force sensor and/or torque sensor.

3. The orthopedic technical device as claimed in one of the preceding claims, **characterized in that** the sensor (30) is arranged on the receptacle (12, 16).

4. The orthopedic technical device as claimed in one of the preceding claims, **characterized in that** the sensor is an angle sensor (30) for registering an inclination with respect to the vertical (V) and for emitting an electrical inclination signal (a_{W}).

5. The orthopedic technical device as claimed in one of the preceding claims, **characterized by**
(a) a thigh receptacle (12) for attachment to a thigh (14),
(b) a shank receptacle (16) for attachment to a shank (18), and
(c) a locking and/or damping mechanism (28) for releasably locking and/or damping a relative movement between thigh receptacle (12) and shank receptacle (16).

6. The orthopedic technical device as claimed in one of the preceding claims, **characterized in that** the electrical evaluation unit (34) has a digital storage medium and is designed to actuate the locking and/or damping mechanism (28) as a function of the inclination signal (a_{w}) in the operating mode for activating and for releasing the locking and/or damping mechanism (28) and to carry out a method with the following steps:
(i) switching into the learning mode,
(ii) establishing the fitting-error characteristic value (R, S, N, a_{W,Schwelle}) that encodes a positional deviation between a correct position of the angle sensor (30) and an actual position of the angle sensor (30'),
(iii) storing the fitting-error characteristic value (R, S, N, a_{W,Schwelle}) in the digital storage medium, and
(iv) activating and releasing the locking and/or damping mechanism (28) with the aid of the fitting-error characteristic value (R, S, N, a_{W,Schwelle}) in the operating mode.

7. The orthopedic technical device as claimed in one of the preceding claims, **characterized in that** the electrical evaluation unit (34) is configured to activate the locking and/or damping mechanism and to establish the fitting-error characteristic value (R, S, N) while the locking and/or damping mechanism (28) locks or are damped the thigh receptacle (12) and the shank receptacle (16) relative to one another.

8. The orthopedic technical device as claimed in claim 7, **characterized in that** the electrical evaluation unit is configured to carry out a method with the following steps:
(i) establishing a fitting-error characteristic value in the form of an inclination-error characteristic value (N) that encodes the minimum value (a_{W,min}) or the maximum value (a_{W,max}) of the electrical inclination signal (a_{w}) after switching into the learning mode, and
(ii) storing the inclination-error characteristic value (N) in the digital storage medium,
(iii) wherein the locking and/or damping mechanism (28) is activated and released on the basis of the inclination-error characteristic value (N) in the operating mode.

9. The orthopedic technical device as claimed in claim 7, **characterized in that** the electrical evaluation unit is configured to carry out a method with the following steps:
(i) establishing a fitting-error characteristic value in the form of a side-error characteristic value (S) that encodes a sign of a change in the inclination signal (a_{w}) when the thigh receptacle (12) swings forward, and
(ii) storing the side-error characteristic value (S) in the digital storage medium,
(iii) wherein the locking and/or damping mechanism (28) is locked and released on the basis of the side-error characteristic value (S) in the operating mode.

10. The orthopedic technical device as claimed in claims 7 to 8, **characterized in that** establishing the fitting-error characteristic value (a_{W,Schwelle}) comprises the following steps:
(i) establishing the maximum value (a_{W,max}) of the inclination signal (a_{W}),
(ii) establishing a threshold (a_{W,Schwelle}), which is smaller than the maximum value (a_{W,max}),
(iii) emitting an opening command to the locking and/or damping mechanism (28) if the inclination signal (a_{W}) drops below the threshold (a_{W,Schwelle}),
(iv) registering whether the locking and/or damping mechanism (28) is released, and
(v) reducing the threshold (a_{W,Schwelle}) if not,
(vi) carrying out steps (ii) to (v) until the locking and/or damping mechanism (28) is released, and
(vii) storing the fitting-error characteristic value in the form of the last threshold (a_{W,Schwelle}).

11. The orthopedic technical device as claimed in claim 9, **characterized in that** the electrical evaluation unit is configured to carry out a method with the following steps:
- establishing the minimum value (a_{W,min}) of the inclination signal (a_{W}),
- storing the last threshold (a_{W,Schwelle}) if the latter is less than 0.9 times an average between maximum value (a_{W,max}) and minimum value (a_{W,min}).

12. The orthopedic technical device as claimed in claim 9 or 10, **characterized in that** the electrical evaluation unit (34) is configured to carry out a method with the following steps:
- establishing whether the locking and/or damping mechanism (28) is released during a predetermined number (N) of opening commands, and
- carrying out steps (ii) to (v) as per claim 13 for an unchanging threshold (a_{W,Schwelle}) until the locking and/or damping mechanism (28) is released during the predetermined number (n_{G}) of opening commands, before the last threshold (a_{W,Schwelle}) is stored.

13. A method for configuring an orthopedic technical device as claimed in one of the preceding claims, comprising the following steps:
(i) switching into a learning mode,
(ii) moving the orthopedic technical device such that at least one fitting-error characteristic value (R, S, N, a_{W,Schwelle}) can be established,
(iii) entering status information into the evaluation unit (34), which encodes the state that the fitting-error characteristic value (R, S, N, a_{W,Schwelle}) should be stored, and
(iv) entering status information into the evaluation unit (34), which encodes the state that a switch into the operating mode should be undertaken.

14. The method as claimed in claim 13, **characterized by** the steps of:
(a) moving the thigh receptacle (12) into a forward-step position,
(b) entering status information into the evaluation unit (34), which encodes the state that the thigh receptacle (12) is in the forward-step position such that an electrical evaluation unit (34) of the orthosis (10) can establish a maximum value (a_{W,max}) or a minimum value (a_{W,min}) of the inclination signal (aW),
(c) moving the thigh receptacle (12) into a standing position such that the electrical evaluation unit (34) of the orthosis (10) can correspondingly establish the maximum value (a_{W,max}) or the minimum value (a_{W,min}) of the inclination signal (a_{w}),
(d) entering status information into the evaluation unit, which encodes the state that the thigh receptacle (12) is in the standing position, and
(e) entering status information into the evaluation unit (34), which encodes the state that input is complete.

## Revendications

1. Dispositif orthopédique pour les extrémités inférieures, comprenant
(a) au moins un récepteur (12, 16) pour une extrémité (14, 18),
(b) un capteur (30) et
(c) une unité d'évaluation (34) électrique connectée au capteur (30) pour le traitement de données du capteur (30),
**caractérisé en ce que**
(d) l'unité d'évaluation électrique (34) est agencée pour mettre en oeuvre un procédé avec les étapes suivantes :
(i) commutation vers un mode d'apprentissage,
(ii) détermination d'au moins une valeur caractéristique d'erreur de montage (R, S, N, a_{W,Schwelle}) qui code une erreur de montage du capteur (30),
(iii) mémorisation de la valeur caractéristique d'erreur de montage (R, S, N, a_{W,Schwelle}),
(iv) commutation en un mode de service, et
(v) dans le mode de service, correction des données du capteur au moyen de ladite au moins une valeur caractéristique d'erreur de montage (R, S, N, a_{W,Schwelle}).

2. Dispositif orthopédique selon la revendication 1, **caractérisé en ce que** le capteur (30) comprend un capteur angulaire, un capteur de force et/ou un capteur de couple.

3. Dispositif orthopédique selon l'une des revendications précédentes, **caractérisé en ce que** le capteur (30) est agencé sur le récepteur (12, 16).

4. Dispositif orthopédique selon l'une des revendications précédentes, **caractérisé en ce que** le capteur est un capteur angulaire (30) pour détecter une inclinaison par rapport à la verticale (V) et pour délivrer un signal électrique d'inclinaison (a_{w}).

5. Dispositif orthopédique selon l'une des revendications précédentes, **caractérisé par**
(a) un récepteur de cuisse (12) pour la fixation sur une cuisse (14),
(b) un récepteur de jambe (16) pour la fixation sur une jambe (18), et
(c) un dispositif de blocage et/ou d'amortissement (28) pour bloquer et/ou amortir, de manière libérable, un mouvement du récepteur de cuisse (12) et du récepteur de jambe (16) l'un par rapport à l'autre.

6. Dispositif orthopédique selon l'une des revendications précédentes, **caractérisé en ce que** l'unité d'évaluation électrique (34) possède une mémoire numérique et est réalisée pour, en mode de service, piloter le dispositif de blocage et/ou d'amortissement (28) en dépendance du signal d'inclinaison (a_{w}) pour activer et pour libérer le dispositif de blocage et/ou d'amortissement (28) et pour mettre en oeuvre un procédé avec les étapes suivantes :
(i) commutation vers un mode d'apprentissage,
(ii) détermination d'au moins une valeur caractéristique d'erreur de montage (R, S, N, a_{W,Schwelle}) qui code une erreur de montage du capteur (30),
(iii) mémorisation de la valeur caractéristique d'erreur de montage (R, S, N, a_{W,Schwelle}),
(iv) commutation en un mode de service, et
(v) dans le mode de service, correction des données du capteur au moyen de ladite au moins une valeur caractéristique d'erreur de montage (R, S, N, a_{W,Schwelle}).

7. Dispositif orthopédique selon l'une des revendications précédentes, **caractérisé en ce que** l'unité d'évaluation électrique (34) est conçue pour activer le dispositif de blocage et/ou d'amortissement et pour déterminer la valeur caractéristique d'erreur de montage (R, S, N) pendant que le dispositif de blocage et/ou d'amortissement (28) bloque ou amortit le récepteur de cuisse (12) et le récepteur de jambe (16) l'un par rapport à l'autre.

8. Dispositif orthopédique selon la revendication 7, **caractérisé en ce que** l'unité d'évaluation électrique est conçue pour mettre en oeuvre un procédé avec les étapes suivantes :
(i) après la commutation vers le mode d'apprentissage, détermination d'une valeur caractéristique d'erreur de montage sous la forme d'une valeur caractéristique d'erreur d'inclinaison (N), qui code la valeur minimum (a_{W,min}) ou la valeur maximum (a_{W,max}) du signal électrique d'inclinaison (a_{w}), et
(ii) mémorisation de la valeur caractéristique d'erreur d'inclinaison (N) dans la mémoire numérique,
(iii) dans lequel en mode de service le dispositif de blocage et/ou d'amortissement (28) est activé et libéré au moyen de la valeur caractéristique d'erreur d'inclinaison (N).

9. Dispositif orthopédique selon la revendication 7, **caractérisé en ce que** l'unité d'évaluation électrique est conçue pour mettre en oeuvre un procédé avec les étapes suivantes :
(i) détermination d'une valeur caractéristique d'erreur de montage sous la forme d'une valeur caractéristique d'erreur de côté (S), qui code un signe d'une variation du signal d'inclinaison (a_{w}) lors d'une oscillation vers l'avant du récepteur de cuisse (12), et
(ii) mémorisation de la valeur caractéristique d'erreur de côté (S) dans la mémoire numérique,
(iii) dans lequel en mode de service le dispositif de blocage et/ou d'amortissement (28) est bloqué et libéré en fonction de la valeur caractéristique d'erreur de côté (S).

10. Dispositif orthopédique selon l'une des revendications 7 à 8, **caractérisé en ce que** la détermination de la valeur caractéristique d'erreur de montage (a_{w},_{Schwelle}) comprend les étapes suivantes :
(i) détermination de la valeur maximum (a_{W,max}) du signal d'inclinaison (a_{w}),
(ii) détermination d'une valeur seuil (a_{W,Schwelle}) qui est plus petite que la valeur maximum (a_{W,max}),
(iii) envoi d'un ordre d'ouverture au dispositif de blocage et/ou d'amortissement (28) quand le signal d'inclinaison (a_{w}) passe au-dessous de la valeur seuil (a_{W,Schwelle}),
(iv) détection pour savoir si le dispositif de blocage/ou d'amortissement (28) est libéré, et
(v) dans le cas négatif, réduction de la valeur seuil (a_{W,Schwelle}),
(vi) exécution des étapes (ii) à (v) jusqu'à ce que le dispositif de blocage et/ou d'amortissement (28) soit libéré, et
(vii) mémorisation de la valeur caractéristique d'erreur de montage sous la forme de la dernière valeur seuil (a_{W,Schwelle}).

11. Dispositif orthopédique selon la revendication 9, **caractérisé en ce que** l'unité d'évaluation électrique est conçue pour mettre en oeuvre un procédé avec les étapes suivantes :
- détermination de la valeur minimum (a_{W,min}) du signal d'inclinaison (a_{W}),
- mémorisation de la dernière valeur seuil (a_{W,Schwelle}) quand cette dernière est inférieure à 0,9 fois une valeur moyenne entre valeur maximum (a_{W,max}) et valeur minimum (a_{W,min}).

12. Dispositif orthopédique selon la revendication 9 ou 10, **caractérisé en ce que** l'unité d'évaluation électrique (34) est conçue pour mettre en oeuvre un procédé avec les étapes suivantes :
- détermination pour savoir si, pour un nombre prédéterminé (N) d'ordres d'ouverture, le dispositif de blocage et/ou d'amortissement (28) est libéré, et
- exécution des étapes (ii) à (v) selon la revendication 10 pour une valeur seuil (a_{W,Schwelle}) qui reste constante, jusqu'à ce que le dispositif de blocage et/ou d'amortissement (28) soit libéré au nombre prédéterminé (n_{G}) d'ordres d'ouverture avant que la dernière valeur seuil (a_{W,Schwelle}) soit mémorisée.

13. Procédé pour organiser un dispositif orthopédique selon l'une des revendications précédentes, avec les étapes suivantes :
(i) commutation vers un mode d'apprentissage,
(ii) déplacement du dispositif orthopédique de telle façon qu'au moins une valeur caractéristique d'erreur de montage (R, S, N, a_{W,Schwelle}) peut être déterminée,
(iii) saisie d'une information d'état dans l'unité d'évaluation (34) qui code la situation où la valeur caractéristique d'erreur de montage (R, S, N, a_{W,Schwelle}) doit être mémorisée, et
(iv) saisie d'une information d'état dans l'unité d'évaluation (34) qui code la situation où il s'agit de commuter vers le mode de service.

14. Procédé selon la revendication 13, **caractérisé par** les étapes suivantes :
(a) déplacement du récepteur de cuisse (12) vers une position correspondant à un pas en avant,
(b) saisie d'une information d'état dans l'unité d'évaluation (34), qui code la situation où le récepteur de cuisse (12) se trouve dans la position correspondant à un pas en avant, de sorte qu'une valeur maximum (a_{W,max}) ou une valeur minimum (a_{W,min}) du signal d'inclinaison (a_{w}) peut être déterminée par une unité d'évaluation électrique (34) de l'orthèse (10),
(c) déplacement du récepteur de cuisse (12) jusque dans une position debout, de sorte qu'un signal d'inclinaison (a_{w}) peut être déterminé par l'unité d'évaluation électrique (34) de l'orthèse (10), en correspondance de la valeur maximum (a_{W,max}) ou de la valeur minimum (a_{W,min}),
(d) saisie d'une information d'état dans l'unité d'évaluation, qui code la situation où le récepteur de cuisse (12) se trouve dans la position debout, et
(e) saisie d'une information d'état dans l'unité d'évaluation (34), qui code la situation où une saisie est terminée.
